(19) Europäisches Patentamt
European Patent Office
Office européen des brevets

(11) **EP 3 564 355 A1**

(12) **EUROPEAN PATENT APPLICATION**

(43) Date of publication:
**06.11.2019 Bulletin 2019/45**

(51) Int Cl.:
***C12M 1/42*** *(2006.01)*     ***C12M 3/06*** *(2006.01)*

(21) Application number: **19172147.1**

(22) Date of filing: **01.05.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.05.2018 US 201862665101 P**

(71) Applicant: **The Charles Stark Draper Laboratory, Inc.**
**Cambridge, MA 02139-3563 (US)**

(72) Inventors:
- **COPPETA, Jonathan R.**
  **Windham, NH 03087 (US)**
- **TANDON, Vishal**
  **Somerville, MA 02144 (US)**
- **TRUSLOW, James G.**
  **Boston, MA 02210 (US)**

(74) Representative: **Arends, William Gerrit**
**Marks & Clerk LLP**
**15 Fetter Lane**
**London EC4A 1BW (GB)**

(54) **METHOD AND DEVICE FOR EXOSOMES ELECTROPORATION**

(57)     Electroporation is conducted in a system comprising a central fluid stream shielded by fluid streams having different electrical conductivities. The streams can be supported by microchannels. In one example, an inner sheath fluid flow is supported by microchannels at each side of a central microchannel. An outer sheath fluid is supported by outer microchannels at the exterior of the inner sheath fluid flow microchannels.

Fig. 1A

**Description**

RELATED APPLICATIONS

This application claims the benefit under 35 USC 119(e) of U.S. Provisional Application No. 62/665,101, filed on May 1, 2018, which is incorporated herein by reference in its entirety.

BACKGROUND OF THE INVENTION

Many applications in biology, medicine, pharmaceutical research and other areas use techniques in which genetic materials are introduced into cells. The term "transformation" is often used when working with bacteria or non-animal eukaryotic cells, including plant cells. "Transfection" almost always refers to work on eukaryotic cells, while "transduction" typically applies to virus-mediated gene transfer into eukaryotic cells.

Biological materials of interest include not only DNA, siRNA, mRNA, RNP complexes, but also small molecules or proteins such as antibodies. In many cases, the process involves opening transient pores or "holes" in the cell membrane to allow the uptake of the "cargo" material and thus alter or genetically modify the cells.

One common technique used to temporarily permeabilize cells is electroporation. Parameters considered when developing electroporation procedures include cell properties (cell size, shape, membrane structure, surface charge, for example), the cell environment, and attributes of the applied electric field, (e.g., pulse intensity, number of pulses, pulse duration, pulse shape and/or frequency). It is generally believed that membrane permeabilization during electroporation occurs after the applied electric field induces a threshold value in the transmembrane potential or "electroporation threshold" and that, at a given applied electric field, there is a threshold for the number of pulses and pulse length needed for successful electroporation. The Schwan equation and related derivations are often used to estimate a cell's transmembrane potential that develops in response to relevant experimental parameters including applied field, cell size, conductivities of media, cellular cytosol, cell membrane and membrane thickness ("Analytical Description of Transmembrane Voltage Induced by Electric Fields on Spheroidal Cells", Biophysical Journal, Volume 79 August 2000 670-679).

Attractive for potential uses in prognosis, therapy, or as biomarkers, exosomes are cell-derived vesicles that are present in many if not all eukaryotic fluids, including blood, urine, as well as cell culture media. Generally, exosomes are composed of lipid bilayer membranes with multiple adhesive proteins on their surface. Known for their cell-to-cell communication characteristics, it is thought that exosomes may find applications in targeted cell therapy.

Whereas eukaryotic cells typically have a diameter within the range of from about 10 to about 100 microns (μm), typical exosome diameters are between 30 and 100 nanometers (nm). At such reduced dimensions, exosomes are also smaller than most prokaryotic cells (0.1-5.0 μm in diameter).

Various electroporation devices have been developed and are commercially available. However, they are generally designed and/or optimized for prokaryotic and/or eukaryotic cells.

It is generally believed that existing electroporation systems cannot achieve the field strengths thought to be required for exosome electroporation. Although some academic papers (see, e.g., Nucleic Acids Research, 2012, Vol. 40, No. 17 e130, or "Delivery of siRNA to the mouse brain by systemic injection of targeted exosomes", Nature Biotechnology, 20 March 2011, doi:10.1038/nbt.1807) appear to indicate loading of exosomes at low field strengths, it is uncertain that luminal loading was actually achieved in these studies. It is largely thought that luminal loading would protect the therapeutic agent from *in vivo* degradation mechanisms and allow for a higher precision delivery method.

SUMMARY OF THE INVENTION

Attempting to apply existing electroporation approaches to exosomes raises various difficulties. One major impediment relates to physical differences in the relevant length scales for electroporation, with cellular diameters spanning a range several orders of magnitude larger than exosomes. Assuming the Schwan equation ("Analytical Description of Transmembrane Voltage Induced by Electric Fields on Spheroidal Cells", Biophysical Journal, Volume 79 August 2000 670-679) is a valid model of transmembrane potential, exosome equivalent transmembrane potentials are not accessible with commercially available electroporation systems. Specifically, high field strengths of 100-300 kV/cm are required to achieve transmembrane potentials in the 0.2 to 1 Volt range and variable pulse lengths (10 nanoseconds to 1,000 microseconds) may be targeted.

In addition, commercially available electroporation devices expose biologics to direct contact with electrodes, resulting in potential damage due to local heating and Faradaic by-products (hydronium ions, hydroxyl ions, chlorine, free radicals, and electrode breakdown by-products (e.g. aluminum ions and particulate)). Existing electroporation approaches lack microfluidics to transport heat away from thermally susceptible biological entities. They also lack co-localization of exosomes and payload, leading to inefficient use of payload. Absent too is a high throughput of transfection.

A need exists, therefore, for equipment and procedures that target and/or facilitate the electroporation of exosome. A need also exists for approaches that address one or more of the deficiencies discussed above.

Generally, the invention relates to transferring (uploading or unloading) materials into or out of membrane-bound structures such as exosomes, other vesicles, or even cells. In specific aspects, permeabilization of the membrane-bound structures (also generally referred to herein as "vesicles") is conducted by electroporation techniques in a system that includes a central stream containing the membrane-bound structures (vesicles, e.g., exosomes, cells and so forth) and streams containing inner and outer sheath fluids having different electrical conductivities. In one example, the inner sheath fluid is provided via two inner sheath fluid streams, at each side of the central fluid. Two outer sheath fluid streams are disposed at the exterior of the inner sheath fluid streams. In one implementation, the outer sheath fluid has an electrical conductivity that is higher than that of the inner sheath fluid.

Flow patterns can be supported by microchannels and, in some embodiments, the invention relates to a device designed to direct flows such as those described above through an electric field. Additionally, or alternatively, the device provides an individual inlet and corresponding outlet for each flow. In one example, separate inlets/outlets are provided for directing the central flow, a first inner sheath flow, a second inner sheath flow, a first outer sheath flow and a second outer sheath flow.

The device can be assembled, in parallel, for instance, with one or more similar devices in an arrangement that can use common reservoirs and/or other equipment, for increased throughput and efficiency, for example. Furthermore, some aspects of the invention relate to a system that includes at least one device such as described herein, and additional components, e.g., a processor controlling microfluidic flows and electroporation parameters, reservoirs and conduits for supplying or collecting vesicles, buffers, cargo, and so forth.

Practicing the invention facilitates the electroporation of exosomes and addresses problems encountered with conventional systems. Robust, flexible and versatile, embodiments of the invention can be applied or adapted to materials other than exosomes, cells or other vesicles, for instance. Principles described herein also can be employed to remove some or all of the contents held in the membrane-bound structures; that is, opening pores and allowing the internal contents to diffuse out either passively or via active electrophoretic forces. Using a dual sheath arrangement and a combination of electrical conductivities such as described herein reduces voltage requirements, since the voltage drop can be localized to the fluid of interest (exosome/payload fluid(s)).

In general, according to one aspect, the invention features an electroporation method. The method comprises directing a central fluid stream, inner sheath streams at each side of the central fluid stream and outer sheath streams at the exterior of the inner sheath streams through an electric field sufficient to permeabilize membrane-bound structures and allowing cargo to transfer in or out of the membrane-bound structures. At least two of a central fluid, inner sheath fluid and outer sheath fluid have different electrical conductivities.

In embodiments, the membrane-bound structures are contained in the central stream. These structures might be exosomes or cell or other membrane-bound structures.

Preferably, the fluid in the outer sheath streams has an electrical conductivity that is higher than that of the fluid in the inner sheath streams and flow of the streams is maintained in the laminar regime. Further, the flow is preferably supported by microchannels.

Some embodiments include measuring conductivity, temperature, field strength or another property of one or more of the central fluid stream, the inner sheath streams and the outer sheath streams.

In general, according to another aspect, the invention features an electroporation method comprising directing a central fluid stream, an inner sheath stream surrounding the central fluid stream at all sides, and an outer sheath stream surrounding the inner sheath stream at all sides through an electric field sufficient to permeabilize membrane-bound structures and allowing cargo to transfer in or out of the membrane-bound structures.

In general, according to another aspect, the invention features a device comprising microchannels supporting a central fluid stream, inner sheath streams at each side of the central fluid stream and outer sheath streams at the exterior of the inner sheath streams, and electrodes for providing an electric field sufficient to permeabilize membrane-bound structures.

In general, according to another aspect, the invention features a system comprising at least one device including microchannels supporting a central fluid stream, inner sheath streams at each side of the central fluid stream and outer sheath streams at the exterior of the inner sheath streams, and electrodes for providing an electric field sufficient to permeabilize membrane-bound structures and a processor for controlling flow of the central fluid stream, the inner sheath streams and outer sheath streams.

In general, according to a further aspect, the invention features an electroporation method comprising directing a fluid stream that contains exosomes with one or more sheath streams through an electric field sufficient to permeabilize the exosomes, and allowing cargo to transfer in or out of the exosomes, along with a system for effecting this method.

The above and other features of the invention including various novel details of construction and combinations of parts, and other advantages, will now be more particularly described with reference to the accompanying drawings and pointed out in the claims. It will be understood that the particular method and device embodying the invention are shown by way of illustration and not as a limitation of the invention. The principles and features of this invention may be employed in various and numerous embodiments without departing from the scope of the invention.

BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:

FIG. 1A is a schematic view showing a flow arrangement using inner and outer sheath streams.

FIG. 1B is a schematic view of an arrangement configured without sheath flows and remote electrodes.

FIG. 2A and 2B are views of a device configured for a dual sheath flow.

FIG. 3 is a schematic view of an assembly that includes multiple devices configured for dual sheath flow.

FIG. 4 is a schematic diagram of a system including a device configured for a dual sheath flow.

DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

As used herein, the term "and/or" includes any and all combinations of one or more of the associated listed items. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

It will be understood that although terms such as "first" and "second" are used herein to describe various elements, these elements should not be limited by these terms. These terms are only used to distinguish one element from another element. Thus, an element discussed below could be termed a second element, and similarly, a second element may be termed a first element without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The invention generally relates to approaches for transferring one or more material(s), referred to herein as "cargo" or "payload", into or out of membrane-bound structures. Specific aspects relate to microfluidic approaches suitable for the electroporation of vesicles such as exosomes, for instance. Other membrane-bound structures that can be used include various cell types as well as other materials that can be loaded with cargo via electroporation techniques.

The membrane-bound structures can be characterized by their size. Some have a diameter that is less than or equal to about 100 nanometers (nm), e.g., within the range of from about 30 to about 200 nm. Others have a diameter that is within the range of from about 10 to about 100 microns ($\mu$m). In further cases, the membrane-bound structures have a diameter within the range of from about 0.1 to about 5.0 $\mu$m.

In many embodiments the membrane-bound structure is a nanoparticle, i.e., a vesicle having a diameter equal or less than 100 nm, such as, for example, within the range of from about 30 to about 100 nm. In other embodiments, it is a microparticle, e.g., a cell, having a diameter greater than about 100 nm. In many cases, the microparticle can have a diameter of upto 1, 5, 10, 15, 20, 25, 50, 75, or 100 $\mu$m. T-cells, a common type of membrane-bound structures for electroporation transfection, for example, range in size from about 6 $\mu$m to 12 $\mu$m.

The membrane-bound structures can be provided in a suitable buffer, as known in the art or as developed for a particular application. In many instances, the buffer is the vesicles' "preferred" buffer. Examples include but are not limited to commercially available buffers such as BTX Cytoporation T-media and those known in the literature such as "Low-Conductivity Buffers for High-Sensitivity NMR Measurements", J. AM. CHEM. SOC. 2002, 124, 12013-12019; "The influence of medium conductivity on electropermeabilization and survival of cells in vitro", Bioelectrochemistry 54 Z2001. 107-115.

The cargo is a suitable material that can be incorporated into the membrane-bound structures. Examples include but are not limited to small molecules, chromosomes, DNA, RNA, e.g., mRNA, siRNA, gRNA, ssRNA), other genetic materials, oligomers, biomarkers, proteins, transposons, biomolecule complexes, small molecules, therapeutic agents, and so forth. In one illustrative example siRNA is loaded into exosomes as a cancer therapy to knock down oncogenes in vivo.

Cargo to be introduced into the vesicles (exosomes, cells and so forth) can be provided in one or more electroporation buffers or solutions. Many cargo-containing buffers are commercially available. In some cases, a suitable solution can be prepared using, for example, techniques and ingredients known in the art. In some cases, electroporation is conducted while cargo and vesicles are both present in the same medium (e.g., buffer).

Principles described herein can be applied or adapted to the removal of some or all of the contents from the vesicles, e.g., cells or exosomes. In this situation, opening the vesicle pores allows the internal contents to diffuse out either passively or via active electrophoretic forces. Releasing contents from the membrane-bound structures may be particularly useful with cargo that is insufficiently characterized, thus raising potential regulatory or other concerns during therapeutic development.

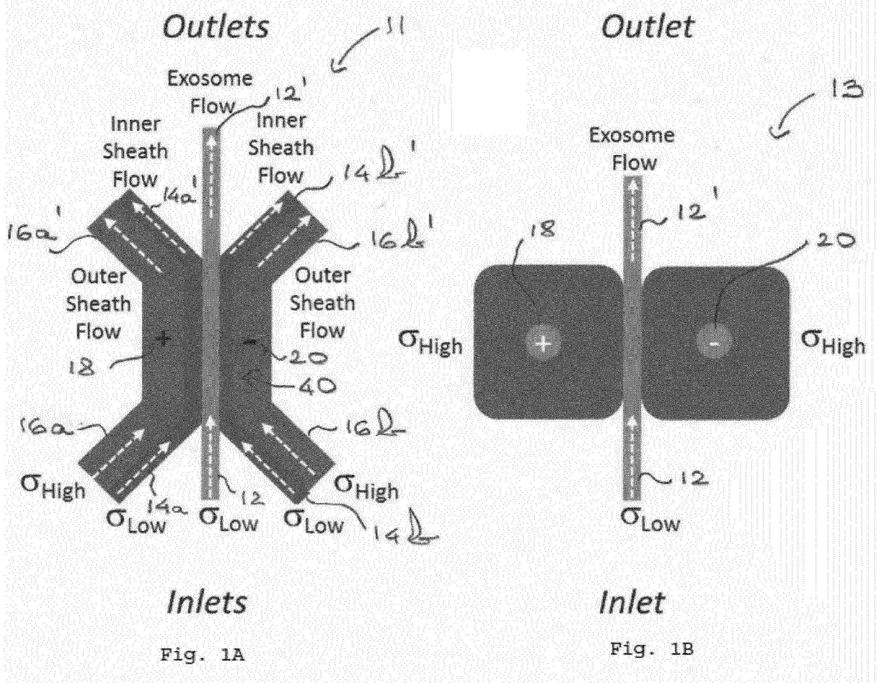

Many of the embodiments described herein use multiple fluid flows (streams). One example includes: a central stream, inner sheath streams at either side of the central stream and outer sheath streams at the exterior boundary of the inner sheath streams. An approach for a dual sheath flow configuration is illustrated in FIG. 1A. In contrast, a design that does not employ a sheath flow configuration is presented in arrangement 13 (FIG. 1B).

Shown in FIG. 1A is arrangement 11 including central stream 12 containing a plurality of membrane-bound structures (exosomes, cells, other vesicles and so forth), inner sheath streams 14a and 14b, outer sheath streams 16a and 16b and a pair of electrodes 18, 20. As seen in FIG. 1A, central stream 12, inner sheath streams 14a, 14b and outer sheath streams 16a, 16b are designed for co-flow travel side by side. Each inner fluid stream 14a, 14b is disposed between the central fluid stream 12 and a corresponding outer fluid stream 16a, 16b, and prevents diffusion from the outer fluid stream into the central fluid stream 12. Other arrangements are possible. One implementation includes concentric flow geometries, with sheath stream 14 and/or 16 surrounding the central stream 12 on all sides.

One approach employs a same inner sheath fluid in both streams 14a and 14b. Similarly, a same outer sheath fluid can be used in both streams 16a and 16b. At least some and typically all of the central, inner sheath and outer sheath fluids have different compositions and/or electrical properties. Stream 12, for instance, can include membrane-bound structures, e.g., exosomes, and payload, in a suitable buffer. For different applications, the central stream 12 carries loaded membrane-bound structures, electroporation being used to promote the removal of cargo from these structures.

In many aspects of the invention, at least two of the fluids employed (e.g., in inner sheath streams 14a, 14b and outer sheath streams 16a, 16b) are characterized by different, e.g., low and high, electrical conductivities (σ), respectively. Examples of suitable fluids for use in outer sheath streams 16a, 16b include but are not limited to salt solutions of NaCl, KCl, NaSO4, KSO4 or H2PO2/HPO4. In specific implementations, the inner sheath fluid has a conductivity that is lower than that of the outer sheath fluid.

In further implementations, the fluid constituting stream 12 also is characterized by a low electrical conductivity. The conductivity of the central fluid (stream 12) and that of the inner sheath fluid (streams 14a, 14b) can be the same, substantially the same, or different.

Suitable conductivity values for the central fluid can be within the range of from about 0.1 to about 0.01 S/m (Siemens per meter). The inner sheath fluid can have a conductivity within the range of from about 0.01 to about 0.1 S/m, while the outer sheath fluid can have a conductivity within the range of from about 1 to about 20 S/m.

Low conductivity fluids allow for high field strengths across the exosome/payload carrying solutions while the high conductivity fluids allow the field lines to spread uniformly with a minimal voltage drop. Employing a combination of conductivities such as described herein reduces voltage requirements, since the voltage drop can be localized to the fluid of interest (exosome/payload fluid(s)).

Exosomes or other vesicles pass between a pair of electroporation electrodes 18, 20 disposed, for example, in zone (region) 40 that focusses the flows. Maintaining laminar flow regimes in focusing region 40 reduces or minimizes mixing between the central and the inner sheath streams and/or the inner and outer sheath streams.

The electrode area (especially the dimension along the flow axis of the channel) and fluid flow rate determine the residence time of the membrane-bound structures in the electric field. Chosen residence times in region 40 can vary from 100 microseconds (μs) to about one second (s). Alternatively, "remote electrodes" can be used, comprising fluidic connections from open ports to the central channel, and wire electrodes placed in the ports.

An AC electric field (for example, sinusoids or pulse trains with periods/pulse widths ranging from 10 nanoseconds (ns) to 100 μs) or a DC electric field is established and remains active while membrane-bound structures flow through the device, and in particular through region 40. The magnitude of the field is tuned for the specific type of membrane-bound structure to a value sufficient to achieve permeabilization. In specific examples, the field is in the range of 100-30,000 kV/m.

Sheath flows can be employed to effect hydrodynamic focusing of individual flows, thus providing a mechanism to adjust the local field strengths operationally by adjusting flow rates. For example, an increased field strength across the biological flow can be achieved by reducing the width of the flow normal to the electric current flow via hydrodynamic focusing.

In addition, the low conductivity fluid employed minimizes Joule heating, an important consideration for biologics. The temperature rise due to joule heating is given by:

$$\Delta T_1 = \frac{\sigma_0 V_0^2 t_p}{\rho c d^2}$$

where $\sigma_0$ is the conductivity, $V_0$ is the applied voltage, $t_p$ is the pulse duration, $p$ is the fluid density, $c$ is the heat capacity and $d$ is the gap between the electrodes. Joule heating in this case is directly proportional to the solution conductivity. Use of microfluidic flow allows convective transport of heat generated via Joule heating.

Another characteristic of some of the microfluidic approaches described herein involves electrodes that are remote from the biological entities. By keeping the electrodes far from the biological entities relative to diffusional length/time scales, potentially damaging faradaic by-products (oxygen, hydronium, chlorine, free radicals) cannot interact with the biological entities nor can the biological entities undergo direct redox reactions at the electrodes.

Specific implementations decouple the electrode geometry from the biological carrying flow stream (central flow stream 12 in FIG. 1A). For example, the microfluidic channels can be designed to compress the electric field lines between the electrodes to allow higher field strengths that are possible with a uniform field while also allowing a more uniform electric field exposure of the biologics.

Providing an inner sheath flow can prevent diffusional or electrophoretic (by the movement of charged particles in a fluid or gel under the influence of an electric field) mixing between the low conductivity biological (central) stream and the outer high conductivity sheath stream, thereby allowing a more uniform field across the biological stream. The flow rates and electrical pulse rates can be matched in designs aimed at minimizing diffusion into the biological stream. For many optimized arrangements, the interaction time of the flow streams is small relative to the diffusion time scales. Or, expressed in a different way, the diffusional length scales would represent a fraction of the inner sheath length scales in the normal field direction.

Furthermore, an inner sheath flow can keep the biological entities away from boundary layer/wall effects, resulting in a more uniform residence time in the area undergoing electroporation.

In many embodiments, the inner sheath flow is unidirectional. However, axisymmetric or sheath flows in the vertical as well has horizontal planes can be utilized, resulting in improved residence time uniformity and thus the anticipated transfection uniformity. Axisymmetric and non-axisymmetric arrangements can be implemented on-chip or via suitably designed tubing from the pumps. For instance, a dual lumen coaxial catheter could be used to create an axisymmetric inner sheath flow surrounding the center biological flow.

For some embodiments, higher field strengths are reached by minimizing the chance of arc nucleating vapor bubbles at the electrodes. This can be accomplished, for example, by minimizing the current required to generate a given field and/or by spatially controlling the field strengths. In some implementations, the current is minimized by using one or more low conductivity fluids to simultaneously minimize gaseous electrolysis products (Faraday's law), while also minimizing Joule heating which can lead to local boiling. Additionally, by minimizing the field strength in the vicinity of the electrodes, any bubbles that do form are less likely to initiate an arc by exceeding the vapor dielectric breakdown voltage (-30 kV/mm in air).

Sensing devices such as sensing electrodes can be placed within the outer, inner, or central flows to make relevant measurements (e.g. conductivity, temperature, field strength, etc.). These electrodes may provide real time feedback to adjust the operational parameters during electroporation. As an example, an RTD (Resistance Temperature Detector) electrode can be placed in the central flow to monitor the temperature excursion of the biologics. A further example may include placing electrodes to measure the conductivity in the inner sheath and central sheath regions near the aperture outlet to allow non-visual alignment of the flows to the intended outlets and to make sure there is not excessive mixing of the flows. Another example may include placing opposing electrodes across the central flow to measure the potential difference thereby estimating the field strength in the central flow.

Shown in FIG. 1B is arrangement 13, including streams 12, 12', electrodes 18 and 20, which can be remote electrodes, e.g., as described above, and no sheath flow configuration. While arrangement 13 may be simpler to implement, it is not ideal for field uniformity, as the biological flow stream lines fan out. This results in a non-uniform cross-section normal to the electric field. Diffusion and electrophoretic effects raise further considerations to be addressed when using a configuration without sheath flow.

Flows such as, for instance, those shown in FIG. 1A can be supported and/or facilitated by conduits and/or channels (also referred to herein as "microchannels), and further aspects of the invention relate to a device and a system configured for carrying out the electroporation flow arrangement described above.

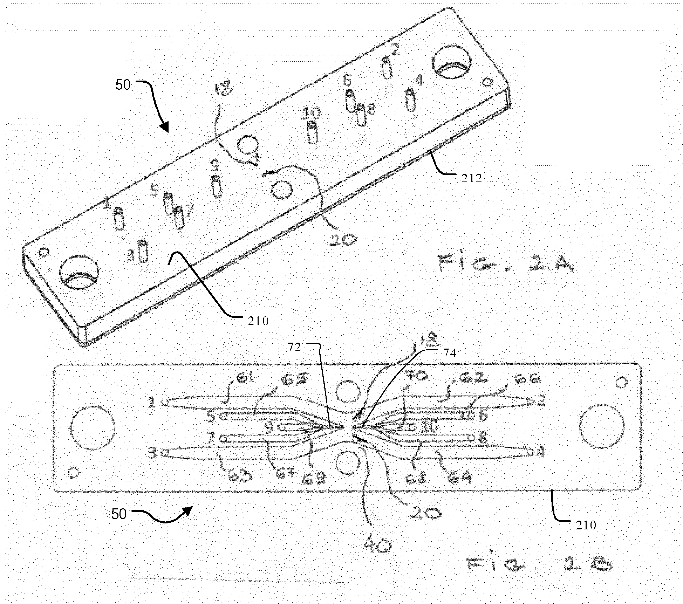

FIGS. 2A and 2B illustrate a prototype embodiment of a device 50 according to aspects of the invention. The device 50 can be constructed as two or three bonded layers or substrates. In one implementation, a top layer or substrate 210 contains microfluidic channels and associated ports, which have been lithographically etched and/or milled into the top substrate 210. The bottom layer or substrate 212 seals the microfluidic channels, thereby creating embedded channels.

The substrates can be made from a polymeric material, e.g., a hard plastic (which, for many materials, renders the device disposable). Examples include but are not limited to COC (cyclic olefin copolymers), such as, for instance, Topas® 6013) or COP (cyclic olefin polymer), Zenor®/Zeonex®) polymers. The two substrates can be thermally adhered using a thin contact zone made, for example from a polymer having a lower glass transition temperature (Tg). One example uses a 25 μm thick contact layer made of 8007 COC. While COC is attractive since it allows optical visualization via microscopy of the flow using an inverted scope, other polymers, glass, silicon, quartz, other ceramics or other suitable materials can be employed to fabricate the device.

Device 50 is configured to include the microchannels that support or guide the various streams described above. In FIGS. 2A and 2B, odd numerals denote inlets to various microchannels, with the corresponding sequential even number referencing the respective outlets. For instance, outlet 2 corresponds to inlet 1. In more detail, inlet channels 1, 3 and respective outlet channels 2, 4 guide outer sheath streams (16a, 16b and 16a' and 16b' in FIG. 1A); inlet channels 5, 7 and corresponding outlet channels 6, 8 guide inner sheath streams (14a, 14b and 14a', 14b' in FIG. 1A); and inlet (input) channel 9 and outlet (output) channel 10 guide a central stream (12 in FIG. 1A).

In more detail, the outer sheath fluid streams 16a, 16b are introduced at inlets 1, 3, and flow through respective conduits (microchannels) 61, 63 that have been fabricated in the substrate 210. Moving in the downstream direction, the outer sheath conduits (microchannels) 61, 63 converge toward each other and open into a focusing zone or region 40 formed in the substrate 210. Then moving further downstream, the peripheral regions of the focusing zone or region 40 empty into conduits (microchannels) 62, 64, which terminate at outlets 2, 4. The exiting outer sheath streams 16a' and 16b' are then withdrawn through these outlets 2, 4.

The inner sheath fluid streams 14a, 14b are provided at inlets 5, 7, and pass through conduits (microchannels) 65, 67 that have been fabricated in the substrate 210. The inner sheath conduits (microchannels) 65, 67 converge toward each other on either side of center conduit (microchannel) 69, which receives the central fluid stream entering at inlet 9. The inner sheath conduits 65, 67 merge with the center conduit 69 to form a short focusing conduit 72, which then empties into the focusing zone or region 40 at the position where the outer sheath conduits 61, 63 also open into the focusing zone or region 40.

On the opposite, downstream side of the focusing zone or region 40 a short downstream center conduit 70 receives the exiting inner sheath fluid stream 14a', 14b' and the exiting center stream 12'.

The short downstream center conduit 70 feeds conduits (microchannels) 66, 68 that diverge away from each other to carry away the exiting inner sheath fluid streams 14a', 14b' to exit at the respective outlets 6, 8. The exiting central fluid stream 12' is carried in exiting center conduit 70 that end at outlets 10.

Inlets 1, 3, 5, 7 and 9 and outlets 2, 4, 6, 8 and 10 can be connected, through suitable conduits, tubing for example, to other components, e.g., reservoirs, provided, for instance for the supply and/or collection of the various streams, as further described below.

Thus, in many of the embodiments described herein, inlets 1 and 3 serve for the introduction of a high conductivity (e.g., 1-20 S/m) outer sheath fluid and are in fluid communication with corresponding outlets 2 and 4. A low conductivity (e.g. 0.01-0.1 S/m) inner sheath fluid is supplied at inlets 5 and 7 and exits the device at corresponding outlets 6 and 8.

The fluid provided to input 9 and exiting at outlet 10 is typically used for the biologic flow stream. In many applications, inlet 9 is used to supply cargo and membrane-bound structures and outlet 10 to direct the removal from the device of cargo-loaded membrane-bound structures. In a different application, cargo-loaded membrane-bound structures are introduced via inlet 9, while released cargo and empty membrane-bound structures leave the device via outlet 10.

Some or all the microchannels can be rectangular in cross section with width and height dimensions that can be within the range of from 100 μm to 2000 μm. The length of the microchannels can be within the range of from about 5 millimeters (mm) to about 30 mm.

These microchannels can be modified or adapted according to the device parameters. Other channels can be added. Furthermore, some implementations provide individual channels that are fabricated separately and are connected fluidically by polymer tubing.

A pair of electroporation electrodes (18, 20) is positioned between the inlet and outlet of streams, halfway between inlet 9 and outlet 10, for example. In some cases, the electrodes are disposed in focused zone (region) 40. In this region, the various streams are in physical contact with one another (for transferring cargo to the vesicles, for instance). Mixing between streams can be prevented, reduced or minimized by maintaining flows in the laminar regime.

The electrode area (especially the dimension along the flow axis of the channel) and the flow rate selected determine the residence time of vesicles in the electric field. Chosen residence times can vary from 100 microseconds (μs) to about a second. It is also possible to use "remote electrodes", such as described above.

Electrodes 18, 20 can be patterned using photolithographic processes onto the polymeric material forming the device. In one example, an electrode is patterned onto the polymer layer with square wire bond or solder pad areas defined by cutouts in the polymer layers that expose the electrodes for external access. Typically, the electrodes are formed from an electrochemically stable material, such as platinum metal (Pt). The portion of the electrodes that are exposed to the fluid in the channels have dimensions of 100-250 μm in width and 8-45 mm in length and interface to a power source via connection to the square soldering pads.

The inner sheath fluid may separate from the biological flow after the electric field aperture (as shown in FIG. 2B), or it may split, together with the outer sheath from the central stream, as in FIG. 1A. In device 50, for example, flow rates may be adjusted to cause the inner sheath to separate at the aperture and travel with the high conductivity outer sheath fluid.

Separating the inner sheath flow from the biological (membrane-bound structures) flow after the electric field aperture may be advantageous in cases where relative conductivities, geometries, and time scales for diffusion create a significant electric leak path across the fluid streams beyond the electric field aperture leading to non-uniform field exposure. This can be modeled and/or predicted using Finite Element Modeling (FEA) or other computational techniques applicable to electric fields and voltages.

Operationally, the pressure and fluid velocities can be nominally matched at the stream interfaces; having separate outlets for each fluid (as shown in FIGS. 2A and 2B) allows the pressure drop to the outlet for each flow stream to be independently tuned to prevent the fluid streams from deforming and/or deflecting. Additionally, ports can create electrical leak paths by providing competing electrical paths with the intended electrical path across the fluid in the electrical aperture.

In some cases, flows can be visualized to allow parameters to be tuned in real time using tracers or imaging the biological entities. Alternatively, electrodes can be placed locally at the aperture beginning and end to allow measurements of the fluid properties (e.g. conductivity or field strength) to optimize the flow parameters in real time such that fluid paths remain aligned to the intended flow paths. This tuning can be passive by designing channel geometries or outlet tubing with the appropriate diameters and lengths or by actively applying pressures using a chamber with a pressure controller.

The device described herein can be part of a system designed to supply and remove ingredients needed to conduct processes related to the uptake or release of cargo into or from membrane-bound structures. In addition to the device, the system includes reservoirs, a processor for controlling various process parameters and, possibly, other elements, e.g., for sensing flow attributes, measuring voltages employed during electroporation, and so forth.

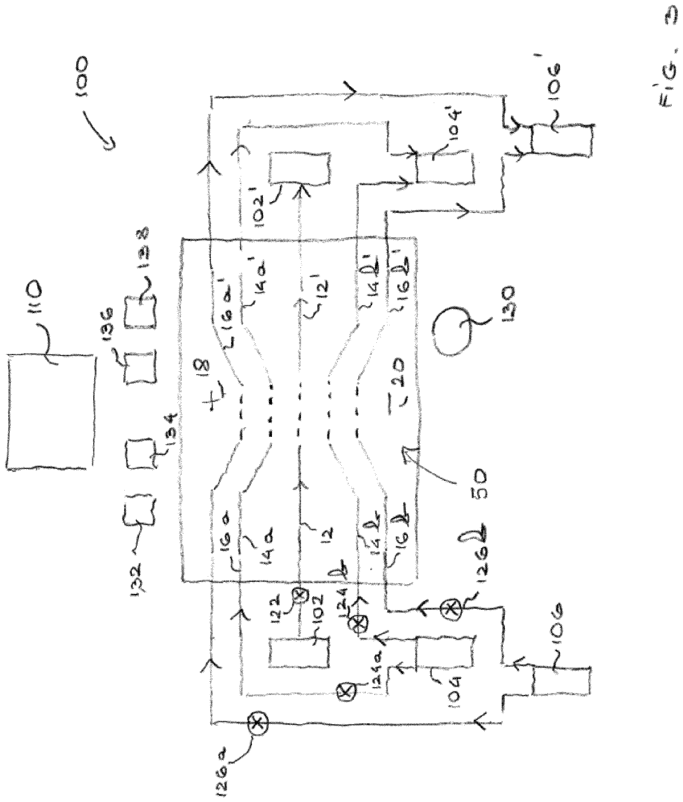

Shown in FIG. 3, for example, is system 100 including a device 50 configured to support a central flow (12, 12'), inner sheath flows (14a, 14a' and 14b, 14b'), and outer sheath flows (16a, 16a' and 16b, 16b'), such as device 50 described with reference to FIGS 2A and 2B; input reservoirs such as reservoirs 102 (supplying the central fluid) 104 (providing the inner sheath fluid) and 106 (supplying the outer sheath fluid); output reservoirs 102' (collecting the central fluid), 104' (collecting the inner sheath fluid) and 106'(collecting the outer sheath fluid); and controller 110. Input reservoirs 102, 104 and/or 106 also can be used to provide sterilizing or washing solutions. In other implementations, washing solution(s) and/or other ingredients can be supplied form additional reservoirs (not shown in FIG. 3). In yet other implementations, membrane-bound structures and cargo are supplied from different reservoirs.

Controller 110 can include computer hardware, software, interfaces and other features that automate the use of device 50 in conducting the transfer of a payload into or out of membrane-bound structures. For instance, controller 110 can initiate, terminate, and/or regulate the flow rates of flows 12, 14a, 14b and 16a, 16b by opening or closing valves 122, 124a, 124b, 126a and/or 126b. On other embodiments, these valves 122, 124a, 124b, 126a and/or 126b are replaced with pumps. In many cases, the valves or pumps are operated to provide side by side flows during the electroporation process. Controller 220 also can initiate electroporation by activating the function generator/voltage source 130.

In some implementations, controller 110 receives measurements from various sensors and uses these measurements to adjust process parameters such as flow attributes (rates, residence time, laminar vs. turbulent profile, alignment of the flow streams on their intended path, etc.) In one example, sensor 132 relies on electrodes suitably placed to allow measurements of the fluid properties (e.g. conductivity or field strength) to optimize the flow parameters in real time of each of the streams 12, 14a, 14b, 16a, 16b, 12', 14a', 14b', 16a', and 16b', individually. Further sensors (134, 136, 138) can be employed to measure stream temperatures, field strengths during electroporation, pressures, and/or other process parameters of each of the streams 12, 14a, 14b, 16a, 16b, 12', 14a', 14b', 16a', and 16b', individually.

Operation of the system can be conducted by an optional initial washing or sterilization of device 50. The controller 22 opens valves or energizes the pumps 122, 124a, 124b, 126a, and 126b to supply central, inner and outer streams 12, 14a, 14b and 16a, 16b to microchannels 61, 63, 65, 67, and 69. Controller 220 also activates the electroporation electrodes 18, 20, and adjusts process parameters based on preset inputs or commands from the operator or on information received from sensors 132, 134, 136, 138. Product is collected at outlet reservoir 102'. Spent inner and outer sheath fluids are collected, respectively, in reservoirs 104' and 106'.

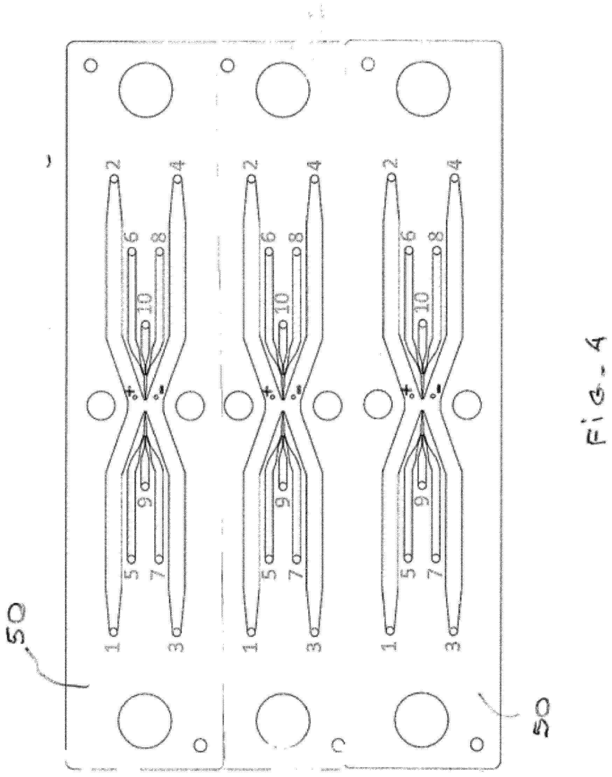

In some implementations, multiple (two or more) microfluidic arrangements such as shown in FIGS 1A and 2B are assembled in parallel, e.g., to draw input solutions from common supply reservoirs. Shown in FIG. 4, for example, is assembly 80 including three arrangements such as described with reference to FIGS 1A and 2B. Assembly 80 can be constructed using a common bottom and a common top plate made from a material, e.g., a hard plastic, such as described above.

Further embodiments described herein relate to mitigating bubble formation via electrolysis products, for example. Various techniques can be employed. One approach relies on degassing the outer sheath fluid e.g., in the region where the electrodes reside, to dissolve or prevent nucleation of gaseous electrolysis by-product.

Further approaches rely on the electrode capacitance (e.g. higher electrode surface area) thereby operating in a capacitive mode and minimizing or eliminating Faradaic current. In turn, electrode surface area can be expanded by techniques such as: increasing electrode nominal size; increasing electrode effective electrochemical area by roughening the substrate; depositing nanoclusters in vapor phase; or by electrochemically depositing rough films (e.g. platinum black).

Yet other implementations employ a porous hydrophobic membrane, fabricated from a suitable hydrophobic material, to promote formed gas bubbles to selectively leave the system, while keeping the fluid in the flow channels.

Bubbles also can be controlled by using PEDOT (PSS or poly(3,4-ethylenedioxythiophene) polystyrene sulfonate) or other conducting polymer or metal (e.g., Ag) that undergoes Faradaic charge transfer.

Also possible is the hydrodynamical separation of the electrode-containing flow from the other flows while maintaining electrical communication. This could be achieved by using conducting hydrogels or a dialysis material. In one implementation, in situ UV polymerization of polyacrylamide, could be utilized to create a physical barrier between the electrode and the sheath flows to prevent bubbles from perturbing the sheath flows while maintaining electrical contact.

Implementations of the invention can be practiced or adapted to reagent-based methods such as delivery by lipids (e.g. transfectamine), calcium phosphate precipitation, cationic polymers techniques, DEAE-dextran, magnetic beads, and virus-based approaches.

While this invention has been particularly shown and described with references to preferred embodiments thereof, it will be understood by those skilled in the art that various changes in form and details may be made therein without departing from the scope of the invention encompassed by the appended claims.

**Claims**

1. An electroporation method, comprising:

directing a central fluid stream, inner sheath streams at each side of the central fluid stream and outer sheath streams at the exterior of the inner sheath streams through an electric field sufficient to permeabilize membrane-bound structures; and

allowing cargo to transfer in or out of the membrane-bound structures,
wherein at least two of a central fluid, inner sheath fluid and outer sheath fluid have different electrical conductivities.

2. The method of claim 1, wherein the membrane-bound structures are contained in the central stream.

3. The method of any of claims 1 and 2, wherein the fluid in the outer sheath streams has an electrical conductivity that is higher than that of the fluid in the inner sheath streams.

4. The method of any of claims 1-3, wherein flow of the streams is supported by microchannels.

5. The method of any claims 1-4, wherein flow rates and electrical pulse rates are configured to minimize diffusion into the central stream.

6. The method of any of claims 1-5, wherein the streams are focused in a region comprising a pair of electrodes.

7. The method of any of claims 1-6, further comprising measuring conductivity, temperature, field strength or another property of one or more of the central fluid stream, the inner sheath streams and the outer sheath streams.

8. An electroporation method comprising

directing a central fluid stream, an inner sheath stream surrounding the central fluid stream at all sides, and an outer sheath stream surrounding the inner sheath stream at all sides through an electric field sufficient to permeabilize membrane-bound structures; and
allowing cargo to transfer in or out of the membrane-bound structures,
wherein fluids in at least two of said streams have different electrical conductivities.

9. A device comprising microchannels supporting a central fluid stream, inner sheath streams at each side of the central fluid stream and outer sheath streams at the exterior of the inner sheath streams, and electrodes for providing an electric field sufficient to permeabilize membrane-bound structures.

10. The device of claim 9, wherein the microchannels are focused in a region containing the electrodes.

11. The device of claim 9 or 10, further comprising one or more sensors for measuring temperature, conductivity, electric field or another property of one or more of the central fluid stream, the inner sheath streams and the outer sheath streams.

12. A system comprising:

at least one device including microchannels supporting a central fluid stream, inner sheath streams at each side of the central fluid stream and outer sheath streams at the exterior of the inner sheath streams, and electrodes for providing an electric field sufficient to permeabilize membrane-bound structures; and
a processor for controlling flow of the central fluid stream, the inner sheath streams and outer sheath streams.

13. The system of claim 12, further comprising input and/or output reservoirs.

14. An electroporation method, comprising:

directing a fluid stream containing exosomes with one or more sheath streams through an electric field sufficient to permeabilize the exosomes; and
allowing cargo to transfer in or out of the exosomes.

15. A system to perform the method of claim 14.

Fig. 1A

Fig. 1B

FIG. 2A

FIG. 2B

FIG. 3

Fig. 4

Europäisches
Patentamt

European
Patent Office

Office européen
des brevets

## EUROPEAN SEARCH REPORT

**Application Number**

EP 19 17 2147

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/176764 A1 (CYTEQUEST INC [US]) 12 October 2017 (2017-10-12) * paragraphs [0025], [0026], [0062]; claim 1; figure 2 * ----- | 1-15 | INV. C12M1/42 C12M3/06 |
| A | US 2012/276635 A1 (LU CHANG [US] ET AL) 1 November 2012 (2012-11-01) * claim 1 * ----- | 1-15 | |
| A | WO 2017/040995 A1 (UNIV RUTGERS [US]) 9 March 2017 (2017-03-09) * claim 1; figure 3b * ----- | 1-15 | |
| A | DATABASE WPI Week 201817 Thomson Scientific, London, GB; AN 2018-067829 XP002794254, -& CN 107 583 676 A (UNIV DALIAN MEDICAL) 16 January 2018 (2018-01-16) * abstract * ----- | 1-15 | |

**TECHNICAL FIELDS SEARCHED (IPC)**

C12M
B01L

The present search report has been drawn up for all claims

| Place of search | Date of completion of the search | Examiner |
|---|---|---|
| Berlin | 16 September 2019 | Jones, Laura |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 17 2147

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2019

| Patent document cited in search report | Publication date | Patent family member(s) | Publication date |
|---|---|---|---|
| WO 2017176764 A1 | 12-10-2017 | CA 3019814 A1<br>EP 3440186 A1<br>US 2017283761 A1<br>WO 2017176764 A1 | 12-10-2017<br>13-02-2019<br>05-10-2017<br>12-10-2017 |
| US 2012276635 A1 | 01-11-2012 | US 2007105206 A1<br>US 2012276635 A1 | 10-05-2007<br>01-11-2012 |
| WO 2017040995 A1 | 09-03-2017 | AU 2016315883 A1<br>CA 2997435 A1<br>CN 109153956 A<br>EP 3344746 A1<br>JP 2018529333 A<br>KR 20180042422 A<br>US 2018258379 A1<br>WO 2017040995 A1 | 29-03-2018<br>09-03-2017<br>04-01-2019<br>11-07-2018<br>11-10-2018<br>25-04-2018<br>13-09-2018<br>09-03-2017 |
| CN 107583676 A | 16-01-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62665101 A

**Non-patent literature cited in the description**

- Analytical Description of Transmembrane Voltage Induced by Electric Fields on Spheroidal Cells. *Biophysical Journal,* August 2000, vol. 79, 670-679
- *Nucleic Acids Research,* 2012, vol. 40 (17), e130
- Delivery of siRNA to the mouse brain by systemic injection of targeted exosomes. *Nature Biotechnology,* 20 March 2011
- Low-Conductivity Buffers for High-Sensitivity NMR Measurements. *J. AM. CHEM. SOC.,* 2002, vol. 124, 12013-12019
- The influence of medium conductivity on electropermeabilization and survival of cells in vitro. *Bioelectrochemistry,* 2001, vol. 54, 107-115